# EUROPEAN PATENT SPECIFICATION

(11) **EP 2 226 099 B1**
(45) Date of publication and mention of the grant of the patent: **08.08.2012**
(21) Application number: 08757476.0
(22) Date of filing: 20.06.2008
(51) Int. Cl.: A61N 7/00, A61B 8/00, G10K 11/00

(54) **AN ULTRASOUND THERAPY HEAD**
ULTRASCHALLTHERAPIEKOPF
TÊTE DE THÉRAPIE À ULTRASONS

(30) Priority: 26.12.2007 CN 200710160630
(43) Date of publication of application: 08.09.2010
(73) Proprietor: Chongqing Ronghai Medical Ultrasound Industry Ltd., Yubei District Chongqing 401121 (CN)
(72) Inventor: HU, Diyuan, Chongqing 401121 (CN); JING, Li, Chongqing 401121 (CN); MAO, Aihua, Chongqing 401121 (CN); ZHAO, Chunliang, Chongqing 401121 (CN)
(74) Representative: Cohausz & Florack
(86) International application number: PCT/CN2008/001204
(87) International publication number: WO 2009/082874

(56) References cited:
- EP-A1- 1 837 052
- CN-A- 1 426 819
- CN-A- 1 765 329
- CN-A- 1 820 712
- CN-A- 1 990 061
- US-A1- 2004 039 312
- US-B1- 6 177 755

## Description

### Field of the Invention

The present invention belongs to the field of ultrasonic therapy, and particularly relates to an ultrasonic therapy applicator.

### Background of the Invention

In the technical field of traditional ultrasonic therapy, an ultrasonic transducer in a therapy applicator generates a great amount of heat during use of the therapy applicator. If the heat is not dissipated in time, a patient may be burned, and the life span of the therapy applicator, particularly the life span of the ultrasonic transducer is influenced.

Generally, in order to prevent the temperature of the therapy applicator from overhigh, it is necessary to use the ultrasonic transducer with reduced power, or at regular intervals to cool down the transducer. In order to solve the above problem, a water cycling cooling system is equipped to the therapy applicator, for instance, an ultrasonic therapeutic instrument in a Chinese Patent (Patent No.: ZL 01144259.X), the circulating water therein serves as a cooling liquid to take away the heat generated by the transducer and serves as an ultrasonic coupling agent functioning to perform ultrasonic coupling as well. However, the water cycling cooling system has the following disadvantages: the pipeline is liable to be aged and damaged, it is hard to be maintained, and it has a large volume, etc. Moreover, sealing the circulating water is still an unsolved problem for the field of ultrasonic therapy all the while. Documents EP 1837052 and US 2004/039312 disclose air-cooled ultrasonic systems.

### Summary of the Invention

Aiming at the above deficiencies existing in the prior art, the technical problem to be solved in the present invention is to provide an ultrasonic therapy applicator which can make the heat generated by the ultrasonic transducer dissipate in time without using any circulating liquid as a cooling medium.

The technical solution for solving the technical problem in the present invention is that said ultrasonic therapy applicator comprises a housing, the housing houses an ultrasonic transducer. There is an opening at one end of the housing, and the opening is sealed by an energy conducting window, the ultrasonic wave from the ultrasonic transducer is emitted outward through the energy conducting window. Wherein, the housing has an air inlet and an air outlet, so that a cooling cavity is formed by a cavity between the air inlet and the air outlet, and the heat produced by the transducer in operation is discharged from the air outlet through the cooling cavity.

In the present invention, said cooling cavity forms between the air inlet and the air outlet in the housing.

Said ultrasonic transducer may be a planar ultrasonic transducer or a spherical ultrasonic transducer. The opening of the housing is sealed by the energy conducting window which has a smooth surface and is made of acoustically transparent material.

A cooling unit is provided between the bottom of the housing and the ultrasonic transducer. Said cooling unit for dissipating the heat produced by the transducer does not employ the circulating liquid, and it may employ various forms, two preferable forms of which are shown as follows:

One form lies in that said cooling unit employs an axial flow fan which is mounted on the back side of an emitting surface of said ultrasonic transducer.

The air outlet and the air inlet are both provided on a side wall of said housing, the air outlet is provided in the vicinity of the opposite side to the emitting surface of said ultrasonic transducer, and the air inlet is provided away from the air outlet. More preferably, a plurality of the air inlets and a plurality of the air outlets can be employed respectively. Suction of cold air and discharge of hot air are performed by the air inlet and the air outlet respectively when the axial flow fan is used, so that an inlet channel and an outlet channel relatively independent from each other are formed inside the ultrasonic therapy applicator.

Another form lies in that said cooling unit includes a refrigerator and a radiator connected in sequence. Said refrigerator is provided at the back side of the emitting surface of said ultrasonic transducer for cooling said ultrasonic transducer, and said radiator is used for performing heat dissipation for the refrigerator. A backing made of heat conducting material is preferably provided between said ultrasonic transducer and the refrigerator. For the spherical ultrasonic transducer, since it can not be kept contact with the refrigerator completely due to its shape, the backing can be provided between the ultrasonic transducer and the refrigerator. The upper and lower surfaces of the backing are kept adaptive contact with the surfaces of the ultrasonic transducer and the refrigerator respectively to cool the backing effectively by using the refrigerator. For the planar ultrasonic transducer, it can be kept direct contact with the refrigerator without the backing; however, the backing can conduct the cooling effect of the refrigerator better. Heat-conducting silicon grease is preferably applied between the ultrasonic transducer and the backing, between the backing and the refrigerator, and between the refrigerator and the radiator (when no backings are used, the heat-conducting silicon grease is applied between the ultrasonic transducer and the refrigerator, and between the refrigerator and the radiator).

Wherein, said backing is made of copper material, and said refrigerator is a semiconductor refrigerator. Said radiator is made of metal material having high heat conduction performance.

More preferably, an axial flow fan is provided along the heat radiation direction of said radiator.

When no axial flow fans are used in the cooling unit, the air inlet is not distinguished from the air outlet obviously, and the hot air exchanges with the external air through each air inlet and/or air outlet. When the cooling unit has an axial flow fan, the suction of cold air and the discharge of hot air are performed by the air inlet and the air outlet respectively, so that an inlet channel and an outlet channel relatively independent from each other are formed inside the ultrasonic therapy applicator. Preferably, a plurality of the air inlets and a plurality of the air outlets can be employed respectively.

Furthermore, a coupling agent can be filled between said ultrasonic transducer and the energy conducting window, so that the ultrasonic wave emitted by the ultrasonic transducer can enter the human body through the energy conducting window. Said coupling agent is an ultrasonic coupling medium processed by vacuum degassing.

The ultrasonic therapy applicator in the present invention does not employ a liquid circulating device as a cooling unit, and it can secure that the heat produced by the ultrasonic transducer can be dissipated in time. The cavity left in the housing serving as the cooling cavity is the channel for heat dissipation, and the cooling unit is provided between the bottom of the housing and the ultrasonic transducer, thus, a patient can be prevented from being burned by the heat produced when the ultrasonic transducer is operated, and the ultrasonic transducer can be used normally in a comparatively long period of time.

### Brief Description of the Drawings

Fig. 1 is a structural schematic diagram of Embodiment 1 in the present invention;
Fig. 2 is a structural schematic diagram of Embodiment 2 in the present invention;
Fig. 3 is a structural schematic diagram of Embodiment 3 in the present invention;
Fig. 4 is a structural schematic diagram of Embodiment 4 in the present invention.

Wherein: 1-housing 11- air outlet 12-airinlet 2- energy conducting window 3- coupling agent 4- ultrasonic transducer 5- signal wire 6- axial flow fan 7- radiator 8- refrigerator 9- backing

### Detailed Description of the Preferred Embodiments

The embodiments of the present invention will be further explained below in detail with reference to the accompanying drawings.

The following embodiments are nonrestrictive embodiments in the present invention:

### Embodiment 1:

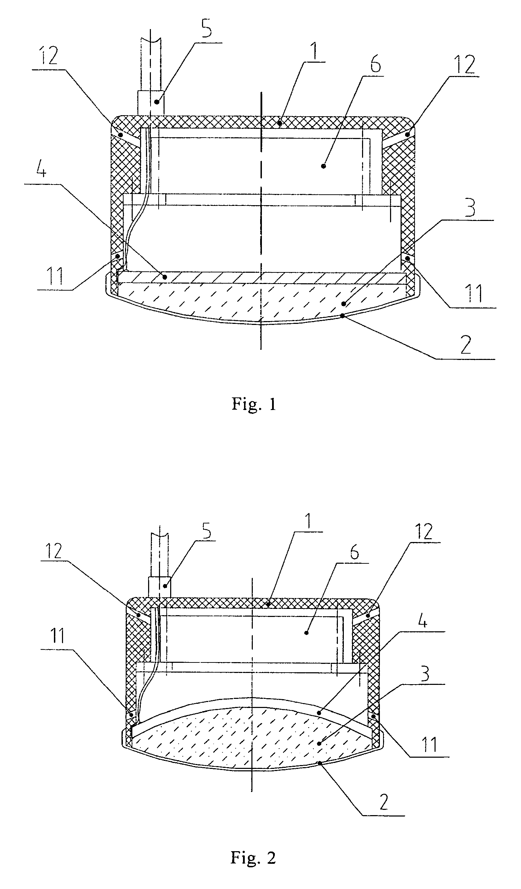

As shown in Fig. 1, an ultrasonic therapy applicator of the present invention comprises a housing 1 in which an ultrasonic transducer 4 and a cooling unit are provided. The housing 1 has a structure which has an opening at the top and has an inner cavity, and the opening is sealed by an energy conducting window 2. The ultrasonic wave from the ultrasonic transducer 4 is emitted outward through the energy conducting window 2.

In this embodiment, the opening of the housing 1 is sealed by the energy conducting window 2 which has a smooth surface and is made of rigid or flexible acoustically transparent material. The ultrasonic transducer 4 adopts a planar ultrasonic transducer which is mounted in the vicinity of the open end of the housing 1, near the energy conducting window 2. An emitting surface of the ultrasonic transducer 4 just faces the energy conducting window 2 to emit ultrasonic wave for therapy.

An air inlet 12 and an air outlet 11 are opened on a side wall of the housing 1. The air inlet 12 sucks the external cold air in to cool the ultrasonic transducer 4, and is provided on the housing 1, adjacent to the bottom of the housing 1; the air outlet 11 discharges the hot air in the housing formed by discharging the heat produced when the ultrasonic transducer 4 is operated into the air, and is provided on the housing 1, in the vicinity of the opposite side to the emitting surface of the ultrasonic transducer 4. A cooling cavity is formed by the cavity between the air inlet 12 and the air outlet 11.

A coupling agent 3 is filled between the energy conducting window 2 and the ultrasonic transducer 4. The coupling agent 3 is made of ultrasonic coupling medium processed by vacuum degassing, and can make the ultrasonic wave for therapy emitted by the ultrasonic transducer 4 successfully reach the treated location.

A signal wire 5 is introduced from the exterior of the housing 1. An outer end of the signal wire 5 is connected to an ultrasonic driver (which is not shown in the figures), and a leading-in end of the signal wire 5 is welded to the ultrasonic transducer 4. The ultrasonic driver drives the ultrasonic transducer 4 to be operated through the signal wire 5.

In the present embodiment, the cooling unit employs an axial flow fan 6 which is provided between the bottom of the housing 1 and the ultrasonic transducer 4, on the back side of the emitting surface of the ultrasonic transducer 4 and near the bottom of the housing 1 for discharging the heat produced by the operation of the ultrasonic transducer 4 from the air outlet 11.

### Embodiment 2:

As shown in Fig. 2, in the present embodiment, the ultrasonic transducer 4 adopts a spherical ultrasonic transducer. Compared with the planar ultrasonic transducer, said spherical ultrasonic transducer can make the ultrasonic waves self-focused.

The structure of other parts in the present embodiment is the same as that of Embodiment 1, and the description thereof is omitted here.

### Embodiment 3:

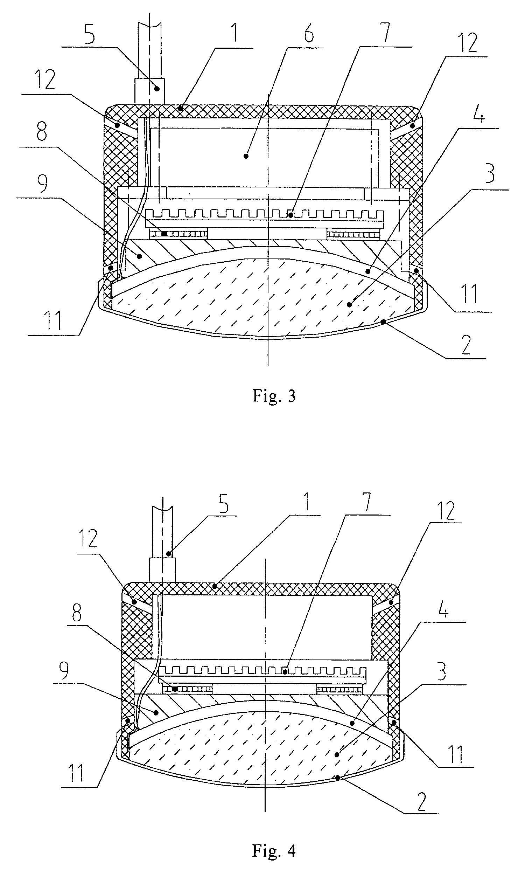

As shown in Fig. 3, in the present embodiment, the ultrasonic transducer 4 adopts a spherical ultrasonic transducer. The cooing unit includes a backing 9, a refrigerator 8, a radiator 7 and an axial flow fan 6. The backing 9 is provided on the back side of the emitting surface of the ultrasonic transducer 4, and the upper and lower surfaces of the backing keep contact with the surfaces of the refrigerator 8 and the ultrasonic transducer 4 respectively. The backing 9 is made of material with high thermal conductivity for conducting the heat from the ultrasonic transducer 4 to the refrigerator 8. The refrigerator 8 made of semiconductor material which clings closely to the face opposite the interface between the backing 9 and the ultrasonic transducer 4 cools the backing 9. The radiator 7 clings closely to the face opposite the interface between the refrigerator 8 and the backing 9 to perform heat dissipation for the refrigerator 8, so that the cooling effect of the whole cooling unit is improved. The axial flow fan 6 is provided on the face opposite the interface between the radiator 7 and the refrigerator 8 and clings closely to the bottom of the housing 1. Heat-conducting silicon grease is applied between the ultrasonic transducer 4 and the backing 9, between the backing 9 and the refrigerator 8, and between the refrigerator 8 and the radiator 7.

During the operation, signals from the ultrasonic driver (which is not shown in the figures) drive the ultrasonic transducer 4 to generate ultrasonic wave for therapy through the signal wire 5. The heat produced by the ultrasonic transducer 4 is conducted to the backing 9, the semiconductor refrigerator 8 cools the backing 9 directly, and the hot air is discharged out of the ultrasonic therapy applicator through the air outlet 11 under actions of the radiator 7 and the axial flow fan 6.

The semiconductor refrigerator 8 employed in the present embodiment can reduce the temperature to 5 °C below the room temperature with accurate temperature control, high reliability, low failure rate, and long service life of more than 200,000 hours. The axial flow fan 6 dissipates heat rapidly.

The ultrasonic transducer 4 can be cooled effectively by employing the cooling unit in the present embodiment, and the use of liquid is avoided in the cooling process.

The structure of other parts in the present embodiment is the same as that of Embodiment 2, and the description thereof is omitted here.

### Embodiment 4:

As shown in Fig. 4, compared with Embodiment 3, the structure of other parts in the present embodiment is the same as that of Embodiment 3 except that the axial flow fan 6 is not used in this embodiment, and the description thereof is omitted here.

Since no axial flow fans 6 are employed in the cooling unit in the present embodiment, the function of the air inlet 12 is not distinguished from that of the air outlet 11 obviously. The hot air can exchange with the external air through the air inlet 12 as well as the air outlet 11.

## Claims

1. An ultrasonic therapy applicator comprising a housing (1) which houses an ultrasonic transducer (4) and has an opening on one end thereof, the opening being sealed by an energy conducting window (2), ultrasonic wave from the ultrasonic transducer being emitted outward through the energy conducting window (2), the housing having an air inlet (12) and an air outlet (11), which are both provided on a side wall of the housing beside the ultrasonic transducer (4), a cooling cavity being formed between the air inlet and the air outlet, heat produced by the ultrasonic transducer (4) in operation being discharged from the air outlet (11) through the cooling cavity, **characterized in that** the air outlet (11) is provided on the side wall near the back side of an emitting surface of the ultrasonic transducer (4), the air inlet (12) is provided on the side wall away from the back side of the emitting surface of the ultrasonic transducer (4) and the air outlet (11) and a coupling agent (3) is filled between said ultrasonic transducer (4) and the energy conducting window (2).

2. The ultrasonic therapy applicator according to claim 1, **characterized in that** a cooling unit is provided between bottom of the housing and the ultrasonic transducer, said cooling unit employing an axial flow fan (6) which is mounted on the back side of the emitting surface of the ultrasonic transducer (4).

3. The ultrasonic therapy applicator according to claim 1, **characterized in that** a cooling unit is provided between bottom of the housing and the ultrasonic transducer, said cooling unit includes a refrigerator (8) and a radiator (7) connected in sequence, said refrigerator (8) is provided on the face opposite the emitting surface of the ultrasonic transducer (4) for cooling the ultrasonic transducer, and said radiator (7) performs heat dissipation for the refrigerator (8).

4. The ultrasonic therapy applicator according to claim 3, **characterized in that** a backing (9) is provided between said refrigerator (8) and the ultrasonic transducer (4), the backing (9) is made of heat-conducting material, upper and lower surfaces of the backing are kept contact with the refrigerator (8) and the ultrasonic transducer (4) respectively, and the backing (9) is cooled by the refrigerator (8).

5. The ultrasonic therapy applicator according to claim 4, **characterized in that** said backing (9) is made of copper material.

6. The ultrasonic therapy applicator according to claim 4, **characterized in that** heat-conducting silicon grease is applied between the ultrasonic transducer (4) and the backing (9), between the backing (9) and the refrigerator (8), and between the refrigerator (8) and the radiator (7).

7. The ultrasonic therapy applicator according to claim 3, **characterized in that** heat-conducting silicon grease is applied between the ultrasonic transducer (4) and the refrigerator (8), and between the refrigerator (8) and the radiator (7).

8. The ultrasonic therapy applicator according to claim 3, **characterized in that** an axial flow fan (6) is provided along heat dissipation direction of said radiator (7).

9. The ultrasonic therapy applicator according to claim 3, **characterized in that** said refrigerator (8) employs a semiconductor refrigerator.

10. The ultrasonic therapy applicator according to claim 1, **characterized in that** said coupling agent employs an ultrasonic coupling medium processed by vacuum degassing.

11. The ultrasonic therapy applicator according to any one of claims 1 to 10, **characterized in that** the ultrasonic transducer (4) employs a planar ultrasonic transducer or a spherical ultrasonic transducer.

12. The ultrasonic therapy applicator according to any one of claims 1 to 10, **characterized in that** the opening of the housing (1) is sealed by the energy conducting window (2) which has a smooth surface and is made of acoustically transparent materials.

## Patentansprüche

1. Ultraschalltherapieapplikator, der ein Gehäuse (1) umfasst, in welchem ein Ultraschallwandler (4) angeordnet ist und welches an einem seiner Enden eine Öffnung aufweist, wobei die Öffnung durch ein Energie leitendes Fenster (2) abgedichtet ist, wobei Ultraschallwellen von dem Ultraschallwandler durch das Energie leitende Fenster (2) hindurch nach außen ausgesendet werden, wobei das Gehäuse einen Lufteinlass (12) und einen Luftauslass (11) aufweist, welche beide an einer Seitenwand des Gehäuses neben dem Ultraschallwandler (4) vorgesehen sind, wobei ein Kühlungshohlraum zwischen dem Lufteinlass und dem Luftauslass ausgebildet ist, wobei von dem Ultraschallwandler (4) während des Betriebs erzeugte Wärme von dem Luftauslass (11) durch den Kühlungshohlraum hindurch abgeführt wird, **dadurch gekennzeichnet, dass** der Luftauslass (11) an der Seitenwand in der Nähe der Rückseite einer emittierenden Fläche des Ultraschallwandlers (4) vorgesehen ist, der Luftauslass (12) an der Seitenwand von der Rückseite der emittierenden Fläche des Ultraschallwandlers (4) und dem Luftauslass (11) entfernt vorgesehen ist und ein Kopplungsmittel (3) zwischen den Ultraschallwandler (4) und das Energie leitende Fenster (2) gefüllt ist.

2. Ultraschalltherapieapplikator nach Anspruch 1, **dadurch gekennzeichnet, dass** eine Kühleinheit zwischen dem Boden des Gehäuses und dem Ultraschallwandler vorgesehen ist, wobei die Kühleinheit einen Axialstromlüfter (6) verwendet, welcher an der Rückseite der emittierenden Fläche des Ultraschallwandlers (4) angebracht ist.

3. Ultraschalltherapieapplikator nach Anspruch 1, **dadurch gekennzeichnet, dass** eine Kühleinheit zwischen dem Boden des Gehäuses und dem Ultraschallwandler vorgesehen ist, die Kühleinheit eine Kühlanlage (8) und einen Radiator (7) aufweist, die in Reihe geschaltet sind, die Kühlanlage (8) auf der der emittierenden Fläche des Ultraschallwandlers (4) gegenüberliegenden Fläche zum Kühlen des Ultraschallwandlers (4) vorgesehen ist und der Radiator (7) eine Wärmeableitung für die Kühlanlage (8) durchführt.

4. Ultraschalltherapieapplikator nach Anspruch 3, **dadurch gekennzeichnet, dass** ein Träger (9) zwischen der Kühlanlage (8) und dem Ultraschallwandler (4) vorgesehen ist, der Träger (9) aus wärmeleitendem Material hergestellt ist, die Oberseite und die Unterseite des Trägers in Kontakt mit der Kühlanlage (8) bzw. dem Ultraschallwandler (4) gehalten werden und der Träger (9) durch die Kühlanlage (8) gekühlt wird.

5. Ultraschalltherapieapplikator nach Anspruch 4, **dadurch gekennzeichnet, dass** der Träger (9) aus Kupfermaterial hergestellt ist.

6. Ultraschalltherapieapplikator nach Anspruch 4, **dadurch gekennzeichnet, dass** ein wärmeleitendes Siliziumschmiermittel zwischen dem Ultraschallwandler (4) und dem Träger (9), zwischen dem Träger (9) und der Kühlanlage (8) sowie zwischen der Kühlanlage (8) und dem Radiator (7) aufgebracht ist.

7. Ultraschalltherapieapplikator nach Anspruch 3, **dadurch gekennzeichnet, dass** wärmeleitendes Siliziumschmierfett zwischen dem Ultraschallwandler (4) und der Kühlanlage (8) sowie zwischen der Kühlanlage (8) und dem Radiator (7) aufgebracht ist.

8. Ultraschalltherapieapplikator nach Anspruch 3, **dadurch gekennzeichnet, dass** ein Axialstromlüfter (6) entlang der Wärmeableitungsrichtung des Radiators (7) vorgesehen ist.

9. Ultraschalltherapieapplikator nach Anspruch 3, **dadurch gekennzeichnet, dass** die Kühlanlage (8) eine Halbleiter-Kühlanlage verwendet.

10. Ultraschalltherapieapplikator nach Anspruch 1, **dadurch gekennzeichnet, dass** das Kopplungsmittel ein Ultraschall-Kopplungsmedium verwendet, das durch Vakuumentgasung bearbeitet wurde.

11. Ultraschalltherapieapplikator nach einem der Ansprüche 1 bis 10, **dadurch gekennzeichnet, dass** der Ultraschallwandler (4) einen planaren Ultraschallwandler oder einen kugelförmigen Ultraschallwandler verwendet.

12. Ultraschalltherapieapplikator nach einem der Ansprüche 1 bis 10, **dadurch gekennzeichnet, dass** die Öffnung des Gehäuses (1) durch das Energie leitende Fenster (2) abgedichtet ist, welches eine glatte Oberfläche aufweist und aus schalldurchlässigen Materialien hergestellt ist.

## Revendications

1. Applicateur de thérapie à ultrasons comprenant un boîtier (1) qui reçoit un transducteur ultrasonore (4) et a une ouverture sur une de ses extrémités, l'ouverture étant hermétiquement fermée par une fenêtre conductrice d'énergie (2), une onde ultrasonore étant émise du transducteur ultrasonore vers l'extérieur à travers la fenêtre conductrice d'énergie (2), le boîtier ayant une entrée d'air (12) et une sortie d'air (11), qui sont toutes les deux fournies sur une paroi latérale du boîtier à côté du transducteur ultrasonore (4), une cavité de refroidissement étant formée entre l'entrée d'air et la sortie d'air, la chaleur produite par le transducteur ultrasonore (4) pendant le fonctionnement étant déchargée de la sortie d'air (11) à travers la cavité de refroidissement, **caractérisé en ce que** la sortie d'air (11) est prévue sur la paroi latérale à proximité du côté arrière d'une surface émettrice du transducteur ultrasonore (4), l'entrée d'air (12) est fournie sur la paroi latérale à l'écart de du côté arrière de la surface émettrice du transducteur ultrasonore (4) et de la sortie d'air (11), et un agent de couplage (3) est rempli entre ledit transducteur ultrasonore (4) et la fenêtre conductrice d'énergie (2).

2. Applicateur de thérapie ultrasonore selon la revendication 1, **caractérisé en ce qu'**une unité de refroidissement est fournie entre le fond du boîtier et le transducteur ultrasonore, ladite unité de refroidissement utilisant un ventilateur axial (6) qui est monté sur le côté arrière de la surface émettrice du transducteur ultrasonore (4).

3. Applicateur de thérapie ultrasonore selon la revendication 1, **caractérisé en ce qu'**une unité de refroidissement est fournie entre le fond du boîtier et le transducteur ultrasonore, ladite unité de refroidissement comprend un réfrigérateur (8) et un radiateur (7) connectés en série, ledit réfrigérateur (8) est fourni sur la face opposée à la surface émettrice du transducteur ultrasonore (4) pour refroidir le transducteur ultrasonore, et ledit radiateur (7) réalise une dissipation thermique pour le réfrigérateur (8).

4. Applicateur de thérapie ultrasonore selon la revendication 3, **caractérisé en ce qu'**un support (9) est fourni entre ledit réfrigérateur (8) et le transducteur ultrasonore (4), le support (9) est formé en un matériau thermoconducteur, les surfaces supérieure et inférieure du support sont maintenues en contact avec le réfrigérateur (8) et le transducteur ultrasonore (4) respectivement, et le support (9) est refroidi par le réfrigérateur (8).

5. Applicateur de thérapie ultrasonore selon la revendication 4, **caractérisé en ce que** ledit support (9) est constitué d'un matériau de cuivre.

6. Applicateur de thérapie ultrasonore selon la revendication 4, **caractérisé en ce que** de la graisse silicone thermoconductrice est appliquée entre le transducteur ultrasonore (4) et le support (9), entre le support (9) et le réfrigérateur (8), et entre le réfrigérateur (8) et le radiateur (7).

7. Applicateur de thérapie ultrasonore selon la revendication 3, **caractérisé en ce que** de la graisse silicone thermoconductrice est appliquée entre le transducteur ultrasonore (4) et le réfrigérateur (8), et entre le réfrigérateur (8) et le radiateur (7).

8. Applicateur de thérapie ultrasonore selon la revendication 3, **caractérisé en ce que** le ventilateur axial (6) est fourni dans le sens de dissipation thermique dudit radiateur (7).

9. Applicateur de thérapie ultrasonore selon la revendication 3, **caractérisé en ce que** ledit réfrigérateur (8) utilise un réfrigérateur à semi-conducteurs.

10. Applicateur de thérapie ultrasonore selon la revendication 1, **caractérisé en ce que** ledit agent de couplage utilise un milieu de couplage ultrasonore traité par dégazage sous vide.

11. Applicateur de thérapie ultrasonore selon l'une quelconque des revendications 1 à 10, **caractérisé en ce que** le transducteur ultrasonore (4) utilise un transducteur ultrasonore plat ou un transducteur ultrasonore sphérique.

12. Applicateur de thérapie ultrasonore selon l'une quelconque des revendications 1 à 10, **caractérisé en ce que** l'ouverture du boîtier (1) est hermétiquement fermée par la fenêtre conductrice d'énergie (2) qui a une surface lisse et est constituée de matériaux acoustiquement transparents.
